# EUROPEAN PATENT APPLICATION

(11) **EP 4 717 140 A1**
(43) Date of publication of application: **01.04.2026**
(21) Application number: 24202543.5
(22) Date of filing: 25.09.2024
(51) Int. Cl.: A61B 1/00, A61B 1/005

(54) **ENDOSCOPE WITH A BENDING SECTION HAVING HINGES**

(71) Applicant: Ambu A/S, 2750 Ballerup (DK)
(72) Inventor: HANSEN, Frederik Clausager Vemb, 2400 Copenhagen NV (DK); JOHNSEN, Lasse Markworth, 3460 Birkerød (DK)
(74) Representative: Winter, Brandl - Partnerschaft mbB

(57) **Abstract**

An endoscope (1) comprising a bending section (20) molded in one piece. The bending section comprises segments (22) connected to each other by hinges (24). An exterior surface (33) of the segments forms an outer surface of the bending section. A central passage (12) is passing each segment and is extending from a distal end to a proximal end of the bending section. Each segment has a recess (18) extending from the exterior surface into the segment. The recesses form a channel (30) extending in parallel with the central passage through the segments. An intermediate hinge and a periphery hinge are connecting two neighboring segments, where the intermediate hinge is placed between the central passage and the channel, and the periphery hinge is placed at the exterior surface of the segments. Electrical cables (7) are extending between the distal tip and the handle are arranged in the channel.

## Description

### Technical Field

The present disclosure relates to an endoscope comprising an insertion cord including a bending section. The bending section is provided with segments being interconnected by bendable hinges.

### Background

Flexible endoscopes for medical purposes are often provided with a bending section at a distal end of an insertion tube. This enables the user of the endoscope to maneuver the distal tip of the endoscope inside the human anatomy, such as in the airways, in the kidneys or the gastro-intestinal system, and e.g., to study or perform procedures at tissue of interest. The bending section is typically bent by pulling steering wires. If the endoscope is a 2-way bending endoscope, i.e., bending in two opposite directions in the same plane, the bending section will typically be controlled by two steering wires, which are controlled by one steering wire actuator arranged in the handle of the endoscope, allowing the user to bend the bending section by adjusting a bending lever. If the endoscope is a four-way bending endoscope, i.e., also bending in two opposite directions in a second plane perpendicular to the first mentioned plane, the bending section will typically be controlled by four steering wires, where one steering wire actuator controls two steering wires for bending in the first plane, and the other steering wire actuator controls the two other steering wires for bending in the second plane.

The steering wires will pass from the handle to the proximal end of the bending section inside wire pipes, which are tubes having limited compressibility. Thereby so-called Bowden cables are formed. The wire pipes are at their proximal end connected inside the handle. At the distal end of the wire pipes, they are connected to the proximal end of the bending section, whereas the steering wires continue through the bending section, often in dedicated lumens. The wire pipes are held stationary and translatory movement of the steering wire at the proximal end is transmitted to the distal end as a corresponding translatory movement of the steering wire relative to the wire pipe.

For single use endoscopes the bending section may be molded in one piece from a polymer material. It is often relevant to provide an insertion cord with the smallest possible outer diameter. Here, the bending section may be a limiting factor. Various factors may be considered when designing a bending section for an endoscope.

One factor is a need for sufficient space for the electrical cables connecting the distal tip and the handle of the endoscope. For a given number of cables, providing more space can enable a reduced bending resistance due to lower frictional forces between the cables during bending. This means that the force which the user has to apply in order to bend the bending section can be lower. Another factor is that a relatively large diameter of the working channel may be preferred. This may allow for larger tools to be applied and for larger flow, e.g. when applying suction through the working channel. Also, another factor is that the design of the bending section, should preferably reduce the risk of kinking of the working channel tube inside the bending section, when the bending section is bent at its maximum bending angle. If the bending section is designed such that the risk of kinking is reduced, it may be possible to apply a thinner wall thickness for the working channel tubing.

### Summary

An object of the present disclosure is to provide an endoscope having a bending section with an optimal compromise between the above-mentioned factors.

In a first aspect, the disclosure provides an endoscope comprising a bending section comprising segments connected to each other by two hinges which are placed opposite one another and adjacent an intermediate passage configured for a working channel tube.

In one embodiment according to the first aspect, the endoscope comprises a handle, and an insertion cord extending from the handle and including a bending section molded in one piece. A camera is arranged in a distal tip distal to the bending section. The bending section comprises:
- Segments connected to each other by hinges, where the hinges allow two neighboring segments to bend relative to each other in a bending plane, an exterior surface of the segments forms an outer surface of the bending section.
- A passage passing each segment and is extending from a distal end to a proximal end of the bending section, where each segment has a circumferential wall, and the circumferential wall has an inner surface facing and encircling the passage.
- A recess extending from the exterior surface of the segments into the segment. The recesses form a channel extending in parallel with the central passage through the segments of the bending section.
- An intermediate hinge and a periphery hinge are connecting each pair of neighboring segments, where the intermediate hinge is placed between the central passage and the channel, and the periphery hinge is placed at the exterior surface of the segments.
wherein electrical cables extending between the distal tip and the handle are arranged in the channel.

Hinges in the bending section molded in one piece of material are parts of the bending section made in dimensions such that the hinges can be bent without plastic deformation of the material.

One effect of this embodiment is that it may enable a smaller outer diameter of the bending section, and at the same time provide a design enabling one or more of: more space for electrical cables, a larger diameter working channel tube, and/or a reduced risk of kinking.

This embodiment may further have the effect of making more space available for the cables so that they are able to slide with a relatively low friction during bending operations of the bending section. This means that the force which the user has to apply in order to bend the bending section can be lower.

The embodiment further has the effect that both hinges will support the working channel and reduce the risk of kinking. Kinking of the working channel has been an issue in the small outer diameter endoscopes having an opening between the working channel lumen and the cable lumen.

In a variation of the present embodiment, lips may be arranged at some or all of the segments. The lips are arranged to extend from a transition between the exterior surface of the segments and the recess, and to follow a circumferential outer periphery of the segment. The lips will thus to some extent cover the channel and reduce the risk of the electrical cables being displaced out of the channel.

In a variation of the present embodiment, at least one segment is provided with a film of material covering the recess. This film is in one piece of material with the bending section, and the film has a thickness smaller than a thickness of a hinge. Such a film on one or more segments has the effect of keeping the electrical cables in place in the channel both during manufacturing, but also during maneuvering the endoscope when an endoscopy procedure is performed.

In a second aspect the disclosure relates to a method for assembling an endoscope, comprising:
providing a bending section molded in one piece from a fused polymer, and having:
   - Segments are connected to each other by hinges, and the hinges allow two neighboring segments to bend relative to each other in a bending plane. An exterior surface of the segments forms an outer surface of the bending section.
   - A passage passing each segment and extending from a distal end to a proximal end of the bending section, where each segment has a circumferential wall, and the circumferential wall has an inner surface facing and encircling the passage.
   - A recess extending from the exterior surface of the segments into the segments, forming a channel extending in parallel with the central passage through the segments of the bending section.
   - An intermediate hinge and a periphery hinge are connecting each pair of neighboring segments, the intermediate hinge is placed between the central passage and the channel, and the periphery hinge is placed at the exterior surface of the segments.
arranging a working channel tube in the passage,
arranging electrical cables in the channel,
arranging a bending cover on the outer surface of the bending section.

This method has been found to be a cost-effective way to manufacture bending sections for endoscopes, e.g., single-use endoscopes, having a small diameter insertion cord. The outer diameter of the insertion cord, including the bending section, may be 3 mm or smaller. The method may also be relevant for larger outer diameters, such as 4 - 6 mm, where advantages such as more space for electrical cables, a larger diameter working channel tube, and/or a reduced risk of kinking of the working channel tube, may be achieved.

In a third aspect the disclosure relates to a system comprising an endoscope according to the first aspect and variations thereof, a monitor and a control unit.

Segments of the bending section include a proximal end segment, a distal end segment and intermediate segments arranged between the proximal end segment and the distal end segment. The segments are not necessarily identical. Especially, the proximal end segment and the distal end segment may have features enabling the connection of the bending section to the insertion tube and to the distal tip housing, respectively.

In this disclosure, the expression "distal" is defined to be in the direction toward the patient, and "proximal" is defined to be in the direction away from the patient. For the handle of the endoscope, the distal end will be the end where the insertion tube is connected, and the proximal end is the opposite end. Further, the expression "handle" may be a positioning interface, or interface, which functions to control the position of the insertion cord and operating the bending section. The handle, or positioning interface, may be an interface operated by a robotic arm, or it may be a handle operated by the hand of an endoscope user.

For this disclosure one steering wire is counted as one passage from the steering wire actuator (or roller) to the distal end of the bending section. I.e., if the same unbroken steering wire continues from the steering wire actuator to the distal end of the bending section and back to the steering wire actuator, and one part is applied for bending for example to one side and the other part is applied for bending for example to the opposite side, this is counted as two steering wires, e.g., first and second steering wires.

### Brief description of the figures

The above-mentioned embodiments and variations, features and advantages thereof will be further elucidated by the following illustrative and nonlimiting detailed description of embodiments disclosed herein with reference to the appended drawings, wherein:
Fig. 1 shows an endoscope and a monitor with a control unit.
Fig. 2 shows an example of a steering wire actuator, here in the form of a roller, and a wire pipe fastener.
Fig. 3A shows a distal end of an endoscope including a bending section.
Fig. 3B shows an enlarged view of part of the bending section in Fig. 3A.
Fig. 4 shows a close-up of the bending section in Fig. 3A viewed from a different angle.
Fig. 5 shows a further close-up of the bending section in Fig. 3A viewed from a different angle.
Fig. 6 shows a cross-sectional view of a segment of a bending section.
Fig. 7 shows a cross-sectional view of a bending section between two segments.
Fig. 8 shows a view similar to Fig. 6, where steering wires, electrical cables and bending cover have been added.
Fig. 9A and Fig. 9B shows two different segments each with a lip extending over the channel, but from opposite sides.
Fig. 10 shows a cross-sectional view of a segment provided with a thin film covering the recess.

In the drawings, corresponding reference characters indicate corresponding parts, functions, and features throughout the several views. The drawings are not necessarily to scale, and certain features may be exaggerated in order to better illustrate and explain the disclosed embodiments. For simplicity, this disclosure will focus on a two-way bending endoscope, but the disclosure is relevant for, and covers, also a four-way bending endoscope.

### Detailed description

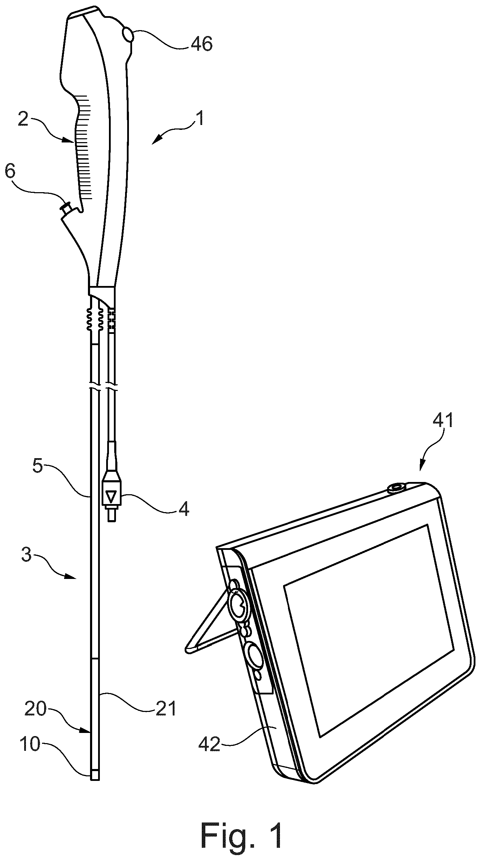

Fig. 1 illustrates an endoscope 1, which comprises a handle 2, an insertion cord 3 and an electrical cable with a connector 4 for connecting the endoscope 1 to a monitor 41. The insertion cord 3 is the part to be inserted into a body lumen during an endoscopic procedure. The insertion cord comprises a distal tip 10, a bending section 20 and a main tube 5. The handle 2 may comprise an entrance to a working channel 6 running through the insertion cord to the distal tip. The handle 2 also comprises a bending lever 46, which can be used for bending the bending section 20. The endoscope handle 2 may be considered as an interface from which the insertion cord 3 extends, and which is configured to control a position of the insertion cord 3. The handle 2 or interface may e.g. be detachably connected to e.g. a robotic arm.

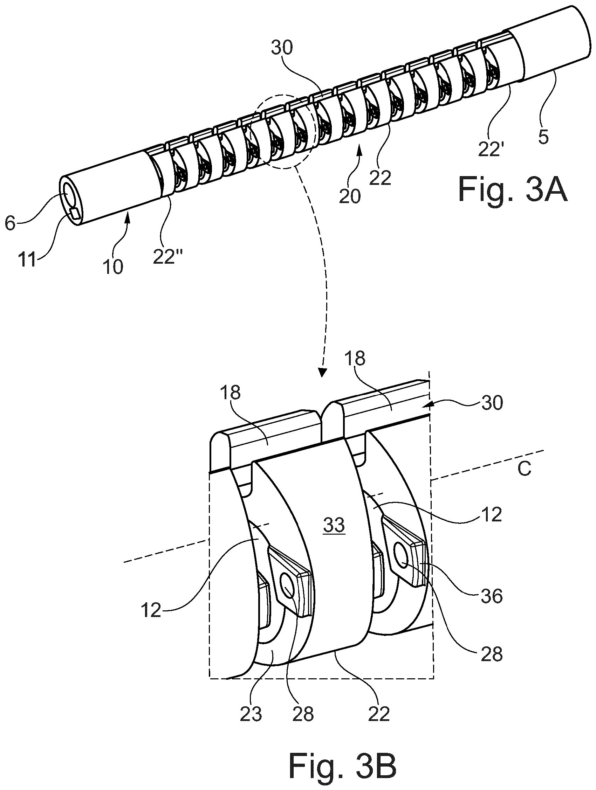

The distal tip 10 comprises a camera 11 (see Fig. 3A) and light emitters (not shown), e.g., in the form of one or more LEDs or the end of an optical light fiber.

The monitor 41 may be combined with an electronic circuit for receiving and processing the image stream from the camera as well as a processor for image processing, user interface, storage of images etc. But the monitor part and the electronic circuit and processor part may also be separate parts. The electronic circuit and the processor part are also referred to as a control unit 42.

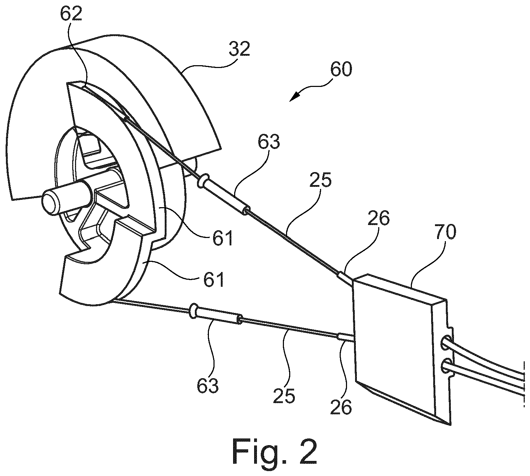

Fig. 2 shows a schematic example of a roller 32 for a steering wire actuator 60 configured for bending the bending section 20 by pulling the steering wires 25. The steering wires 25 are moved by rotation of wire drum curved surfaces 61, which are a circular arc surface or a curved surface placed on the roller 32 and supporting the steering wires 25. In the example, one end of the steering wire 25 has been drawn through a fixing structure 62 and bent back and fastened to itself by a crimp 63. The proximal ends of the wire pipes 26 are fixated in the wire pipe fastener 70, whereas the distal ends of the wire pipes 26 are secured to the proximal end of the bending section 20. Both the wire pipe fastener 70 and the roller 32 are often secured in the handle 2. The system in fig. 2 is for a two-way bending endoscope.

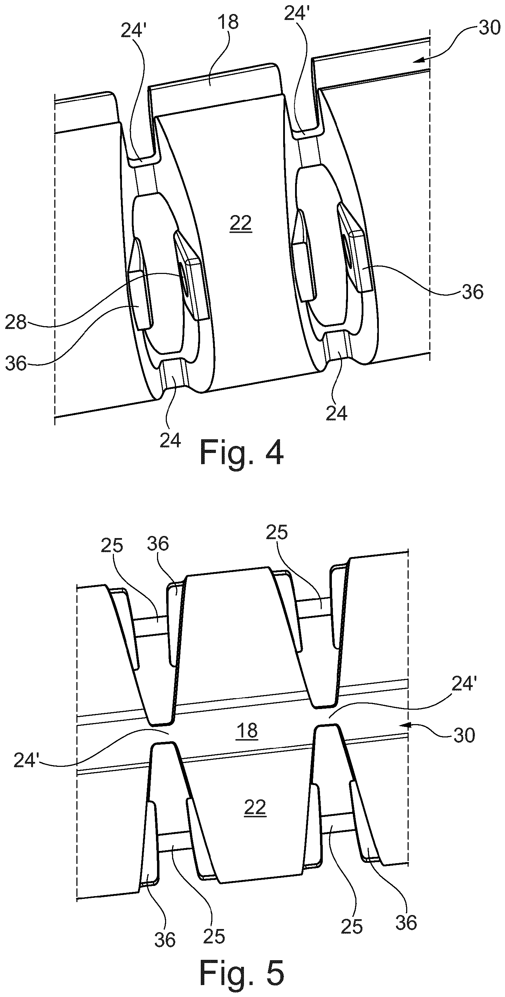

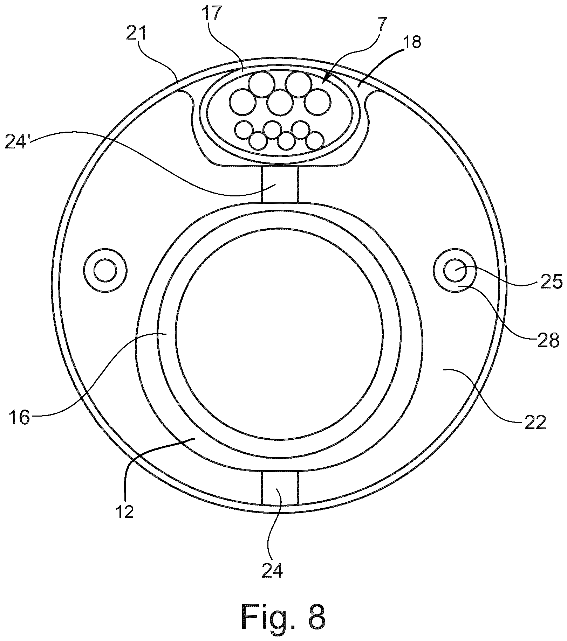

Fig. 3A shows a distal end of the insertion cord 3 of an endoscope, where the bending cover 21 (shown in Fig. 8), typically covering the bending section 20 part and often making this part watertight, has been removed. The proximal end of the bending section 20 is attached to the main tube 5, and the distal end of the bending section 20 is attached to the distal tip 10. In this example the bending section 20 is molded in one piece and comprises a number of segments 22. A proximal end segment 22' is connected to the distal end of the main tube 5. A distal end segment 22" is connected to the distal tip 10. The segments are held together by hinges 24, 24' (see Fig. 4), so that the segments can be bent relative to each other by manipulation of the steering wires 25 (shown in Fig. 5). Another example of an endoscope bending section molded in one piece can be seen in EP 3 925 513 A1, which is incorporated herein by reference in its entirety.

Steering wires 25 are guided in steering wire passages 28 and are connected in a fixed connection to the distal end, e.g., the distal end bending segment 22". I.e., the steering wire 25 is preferably not movable in relation to the distal end bending segment 22". Between the handle and the distal end of the main tube 5, or the proximal end of the bending section 20, the steering wire 25 is guided in a wire pipe 26 which is secured in a wire pipe fastener 70 in the handle 2 distal to the steering wire actuator 60.

The bending section is provided with a central passage 12 extending in parallel with a longitudinal center axis C (see Fig. 3B) of the bending section. The center axis C extends in the direction between the proximal end of the bending section and the distal end of the bending section. A working channel tube 16 (see Fig. 8) may pass through the central passage. The center axis C may be placed in the central passage 12, but not necessarily in the center of the central passage 12.

Fig. 3A and Fig. 3B further shows that the bending section 20 is provided with a channel 30 formed by a number of recesses 18 where each segment 22 of the bending section 20 has a recess 18 extending from an exterior surface 33 of the segment into the circumferential wall 23 of the segment 22. When these recesses 18 are placed on a straight line, when the bending section 20 is straight, they may form the channel 30 extending in parallel with the central passage 12 through the segments 22, 22', 22" of the bending section 20. This channel 30 may be applied for the electrical cables 7 connecting the camera 11 and the light source in the distal tip 10 with a printed circuit board (PCB) in the endoscope handle 2. These electrical cables 7 may be arranged as a bundle, which may be surrounded by a sleeve 17 (see Fig. 8).

Fig. 4 shows an enlarged view of a few bending section segments 22, but from a different angle than the view in Fig. 3B. In Fig. 4 it is possible to see also the hinges 24, 24' connecting two neighboring segments 22. Between two segments 22 there is one periphery hinge 24 and one intermediate hinge 24'. The intermediate hinges 24' are arranged between the central passage 12 and the channel 30. As indicated in Fig. 4 the thickness of the hinges may be similar to the adjacent thickness of the circumferential wall 23 of the segment 22, in a direction radial to the center axis C. The number of hinges between two segments may be exactly two.

Fig. 5 shows a view of a few segments 22, where the bending section 20 is rotated approximately 90 degrees in relation to the view in Fig.4. In Fig. 5 the viewing direction is perpendicular to the center axis C and directly into the channel 30 and the intermediate hinges 24'. In Fig.5 the steering wires 25 have also been added.

Fig. 3B, 4, and 5 also shows bending limiting protrusions 36, which may surround each opening to the steering wire passage 28 in each segment 22. The bending limiting protrusions 36 are protruding from the surfaces of each segments 22 which are facing a corresponding surface of the neighboring segments 22, i.e., the surfaces extending perpendicular to the center axis C. Thereby, the bending limiting protrusions 36 are protruding in a direction parallel to the center axis C. The purpose of the bending limiting protrusions is to define the maximum bending angle of the bending section 20, and to avoid that two neighboring segments 22 squeeze the bending cover 21 between them during bending. Such squeezing of the bending cover 21 could in some situations result in puncture of the bending cover 21.

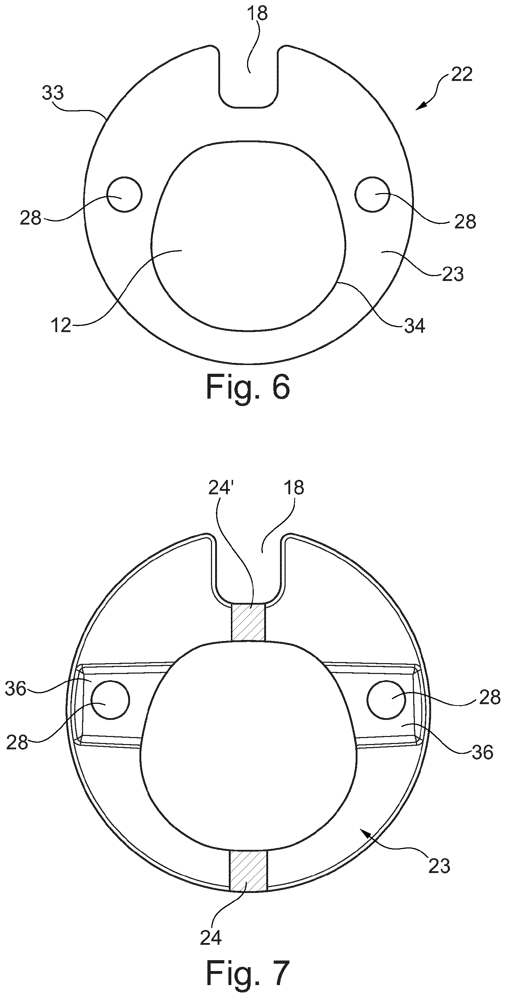

Fig. 6 shows a cross-sectional view of a segment 22 of a bending section 20. The segment 22 comprises an opening for the central passage 12. The central passage 12 passes all segments 22, 22', 22" and is extending from a distal end to a proximal end of the bending section 20. Each segment has a circumferential wall 23, the circumferential wall 23 has an inner surface 34 facing and encircling the passage 12, and an exterior surface 33 encircling the whole segment 22.

The circumferential wall 23 may have a varying wall thickness, where the wall thickness is measured in a radial direction in relation to the center axis C. This wall thickness may be minimum at a position connected to a hinge 24. Here the wall thickness may be similar to the corresponding thickness of the hinge 24. Thereby, the bottom of the channel 30 may be a plane surface, both where the channel 30 passes a segment 22 and where the channel 30 passes over a hinge 24. The thickness of the circumferential wall 23 may have a maximum at a position neighboring a recess 18.

Fig. 6 also shows the recess 18 extending from the exterior surface 33 of the segments 22, 22', 22" into the segments towards the inner surface 34, without reaching the inner surface. The recesses 18 in the segments 22 will thereby form the channel 30 extending in parallel with the central passage 12 through the segments of the bending section.

Fig. 7 shows a cross-sectional view of a bending section between two segments. This view only intersects the hinges, as opposed to fig. 6 intersecting the main part of the segments. By making the cross-sectional view through the hinges 24, 24' only, the bending limiting protrusions 36, surrounding the openings for the steering wire passages 28, becomes visible, which is not the case in Fig. 6. Fig. 7 shows that the thickness, or height, of the hinges 24 in a radial direction to the center axis C may be similar to the wall thickness of the circumferential wall 23.

Fig. 8 shows a cross-sectional view through a segment 22 of a bending section 20, where working channel tube 16, steering wires 25, electrical cables 7 and bending cover 21 has been added. The steering wire passages 28 are shown as closed passages with a circular cross-sectional shape. Other shapes are also possible, and the steering wire passages 28 may also be in open connection with the central passage 12 for the working channel tube 16.

As shown in Fig. 8, the electrical cables 7 may be arranged in a sleeve 17. The bending cover will keep this sleeve 17 with electrical cables 7 in place during bending and manipulation of the endoscope. The assembling of the endoscope may be simpler when the electrical cables 7 are assembled in a sleeve 17 keeping the cables together.

The shape of the segment 22 will provide support for the working channel tube 16 along the full periphery of the tube. This is opposed to solutions where one larger lumen is applied for both the working channel tube 16 and the electrical cables 7. This further support for the working channel tube 16 will reduce the risk of the tube kinking during bending of the bending section. This means that the maximum bending angle of the bending section may be increased, or the bending radius can be decreased at the same bending angle. Also, the wall thickness of the tube material may be reduced, or a softer (more flexible) material may be applied for the working channel tube 16.

Both Fig. 7 and Fig. 8 indicates that the periphery hinge 24 and the intermediate hinge 24' have similar cross-sectional dimensions. However, for some variations the intermediate hinge 24' may be higher (or thicker) than the periphery hinge 24. The height is here measured in a radial direction from the center axis C. A higher intermediate hinge 24' may improve the overall torsional stiffness of the bending section 20. This may compensate for the reduction in torsional stiffness caused by having the intermediate hinge 24' placed closer to the center axis C, compared to a hinge at the outer periphery of the bending section. In an example the intermediate hinge 24' has a height which is at least 25 % larger than the height of the periphery hinge 24. The intermediate hinge 24' may also have a height which is at least 50 % larger than the height of the periphery hinge 24.

It is shown in Fig. 8 that the lumen through the segments for the working channel tube 16 may not have a circular cross-section. This enables having an outer diameter of the working channel tube 16 which is as large as possible, and at the same time still being able to take up variations in the outer diameter of the working channel tube caused by the tolerances on the tube. This means that tubes having the maximum diameter possible within a given tolerance range, can fit into the lumen but may end up having a shape deviating slightly from a circular shape. This is described further in EP 4 233 679 A1 (see e.g. paragraph [0018] and [0062]), which is incorporated herein by reference in its entirety.

As illustrated, the shape of the recesses 18 in the segments differs between the example shown in Fig. 6 and 7, and the example shown in Fig. 8, respectively. The recess 18 may have different shapes and sizes depending on the outer diameter of the bending section 20 and depending on the number of electrical cables 7 necessary for the specific endoscope. The number of electrical cables 7 depend on the type of image sensor in the distal tip 10, and also on the presence of other transducers in the distal tip. Other transducers could for example be temperature or pressure sensors or an ultrasonic transducer. The shape and size of the recess 18 may also, as mentioned above, depend on the need for reducing friction during bending.

For the recess 18 a cross-sectional area A_{R} may be defined. This is the area limited by the inner surface of the bending cover 21, when the bending cover follows a circular shape around the segments 22, and the exterior surface of the segment. Also, a cross-sectional area A_{W} may be defined for the electrical cables 7. This area A_{W} includes the insulation and any shielding of the cables, as well as any sleeve 17 for the electrical cables 7. The relationship between the two areas may have an impact on the friction between the cables 7 and the friction between the cables and the segments 22, when bending the bending section 20, and thereby the bending resistance may also be impacted by this relationship. In one variation the cross-sectional area of the cables Aw is in the range 10 % to 85 % of the cross-sectional area of the recess A_{R}. In one variation Aw is in the range 10 % to 65 % of A_{R}. In one variation Aw is in the range 10 % to 50 % of A_{R}.

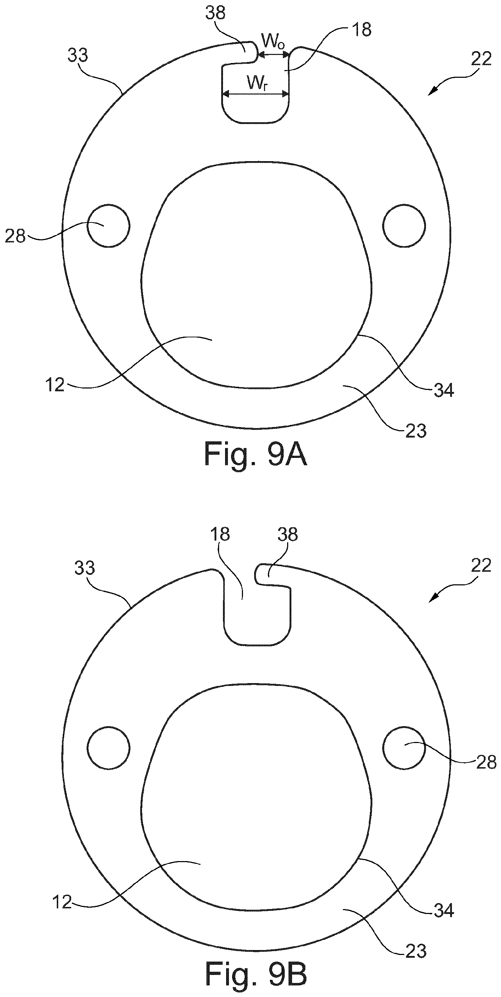

Fig. 9A and Fig. 9B shows two different segments each with a lip 38 extending over the recess 18 forming the channel 30. In Fig. 9A a lip 38 is extending from the left side of the recess in the cross-sectional view, and in Fig. 9B a lip 38 is extending from the right side of the recess in the cross-sectional view. These lips have the effect of keeping the electrical cables 7 inside the channel 30 during bending of the bending section. The bending cover 21 has the same effect, but displacement of the electrical cables may cause some bulging of the bending cover 21, as the bending cover 21 typically has more elasticity than the bending section material. The lips 38 made in the same material, and in one piece with the bending segments 22, will prevent any bulging.

These lips 38 may further have the effect of holding or supporting the electrical cables 7 or a sleeve 17 enclosing the electrical cables 7, in place during manufacturing of the endoscope 1 in the period from arranging the electrical cables 7 in the channel 30 and until the bending cover 21 has been arranged.

As shown in Fig. 9A and 9B the lips 38 have dimensions such that it is still possible to insert a sleeve 17 enclosing the electrical cables 7 through an opening into the channel 30. Also, Fig. 9A shows a different segment 22 of the bending section 20 than Fig. 9B. I.e. in a variation the lips 38 may be connected to opposite sides of the channel 30 for different segments 22. This will reduce the risk that electrical cables 7 may be displaced out of the channel 30 during bending operations.

Lips 38 may be distributed in different patterns along the length direction of the bending section 20. E.g., a lip 38 could be arranged on all or a majority of the segments 22 of the bending section 20. These lips 38 could all extend from the same side of the channel 30, or the lips could extend from one side or from the other side, alternately. A lip 38 could also be arranged on every second or every third or every fourth segment 22 only. Also here, the lips could extend from one side of the channel 30 or from the other side, alternately.

In an example, at least one lip 38 on one segment 22 is extending from a first side of the channel 30, and at least one lip 38 on another segment 22 is extending from a second opposite of the channel 22. In this example, the two segments 22 mentioned may only be provided with one lip 38 each.

In a variation, a segment may be provided with two lips 38 extending from each side of the recess 18 and covering part of the recess 18 and may follow a circumferential outer periphery of the segment 22. The two lips 38 extending over one recess 18 may have different dimensions and extending over a different length of the recess 18 along a circumferential outer periphery of the segment 22.

The lips 38 may have a thickness in a radial direction relative to the center axis C, so that they are sufficiently flexible to be bent slightly during arrangement of the electrical cables 7 into the channel 30. If the bending section 20 is made from POM this thickness could be in the range 0.05 - 0.4 mm, or in the range 0.1 - 0.3 mm. These ranges may also be relevant for polypropylene, but other materials may have other properties in relation to molding capabilities.

The lips 38 may have a length in the direction parallel to the center axis C which is similar to, or equal to the length of the segment 22. The lips 38 may also have a length which is shorter than the length of the segments 22.

The width wᵣ of the recess 18 (see Fig. 9A) forming the channel 30 may be in the range of 0.8 - 3.0 mm depending on e.g. the outer diameter of the bending section 20 and on the number and diameter of electrical cables 7. When a lip 38 is arranged on a segment 22 to partly extend over the recess 18, the lip 38 will result in an opening into the recess 18 having a width wₒ which may be in the range of 0.3 - 2.5 mm. In a variation the width of the opening wₒ may be at least 25 % of the width of the recess wᵣ. Also, the width of the opening wₒ may be at least 40 %, or at least 50 %, or at least 60 % of the width of the recess wᵣ. An upper limit may be that the width of the opening wₒ is at maximum 95 % of the width of the recess wᵣ.

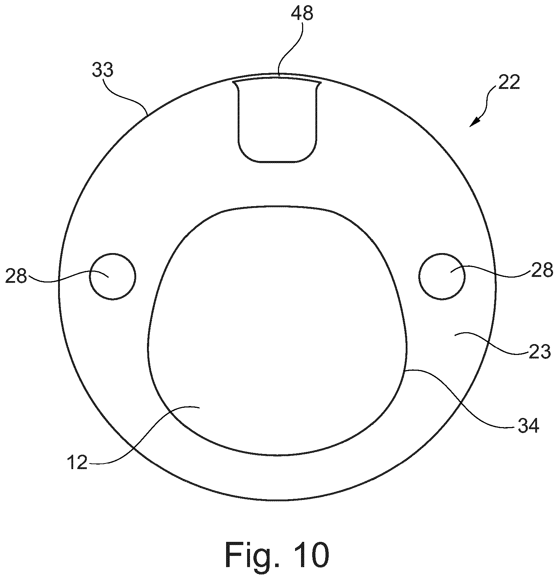

A further variation is shown in Fig. 10, where the segments 22 or some of the segments 22, are provided with a thin film 48 of material covering the recess 18. This thin film 48 supports the electrical cables 7 and keeps them in place but is too thin to function as a hinge. The thin film 48 may be in one piece of material and integral with the bending section 20, e.g., molded in one piece with the bending section 20. Molding in one piece may here be understood to include also a 2K molding process, where e.g. different polymers are fused together. The film 48 may have a thickness smaller than a thickness of a hinge 24, e.g. smaller than half the thickness of a hinge 24.

In an example, the bending section 20 is made from POM and has segments 22 with an outer diameter in the range 3.0 - 6.5 mm, e.g. 5.5 - 6.2 mm, in a cross-sectional view, where the cross-section is made perpendicular to the center axis C. In a similar viewing angle, the hinges 24, 24' have dimensions in the range 0.4 x 0.4 mm to 0.6 x 0.6 mm. The height and the width of the hinges 24, 24' may differ, and the height of the intermediate hinge 24' may be up to up to 0.8 or 0.9 mm. The width wᵣ of the recess 18 may be in the range 1.5 - 3.0 mm, e.g. 2.0 - 2.8 mm.

The height of the channel 30 may be defined in a radial direction relative to the center axis C as the distance between the intermediate hinges 24' and the bending cover 21, when the bending cover 21 follows a circular cross-sectional shape. This height may be in a range of 0.8 - 2.0 mm. The thickness of the bending cover 21 may be in the range of 0.05 - 0.1 mm.

Lips 38 may be arranged to extend over a number of recesses 18 along the length of the channel 30 in the bending section. The lips 38 may be designed to cover 40 - 60 % of the recess 18. If the width wᵣ of the recess is 2.0 mm, the width of the opening wₒ may be 0.8 - 1.2 mm. The outer diameter of the individual electrical cables 7 may be in a range of 0.1 - 0.5 mm, and there may be 3 - 8 cables, e.g. 5 cables. In some cases, there may be 12 - 70 electrical cables 7.

**List of references**

| | | | |
|---|---|---|---|
| 1 | endoscope | 26 | wire pipe |
| 2 | handle | 28 | steering wire passage |
| 3 | insertion cord | 30 | channel |
| 4 | electrical cable with connector | 32 | roller |
| 5 | main tube | 33 | exterior surface |
| 6 | working channel | 34 | inner surface |
| 7 | electrical cables | 36 | bending limiting protrusion |
| 10 | distal tip | 38 | lip |
| 11 | camera | 41 | monitor |
| 12 | central passage | 42 | control unit |
| 16 | working channel tube | 46 | bending lever |
| 17 | sleeve for electrical cables | 48 | thin film |
| 18 | recess | 60 | steering wire actuator |
| 20 | bending section | 61 | wire drum curved surface |
| 21 | bending cover | 62 | fixing structure |
| 22 | segment | 63 | crimp |
| 22' | proximal end segment | 70 | wire pipe fastener |
| 22" | distal end segment | C | center axis |
| 23 | circumferential wall | wᵣ | width of recess |
| 24 | periphery hinge | wₒ | width of opening to recess |
| 24' | intermediate hinge | | |
| 25 | steering wire | | |

## Claims

1. An endoscope comprising:
- a handle;
- an insertion cord extending from the handle and including a bending section molded in one piece, and a camera arranged in a distal tip distal to the bending section, the bending section comprising:
• segments connected to each other by hinges, the hinges allowing two neighboring segments to bend relative to each other in a bending plane, an exterior surface of the segments forms an outer surface of the bending section,
• a central passage passing through each segment and extending from a distal end to a proximal end of the bending section, where each segment has a circumferential wall, the circumferential wall has an inner surface facing and encircling the central passage,
• a recess extending from the exterior surface of the segments into the segments, forming a channel extending in parallel with the central passage through the segments of the bending section,
• an intermediate hinge and a periphery hinge connecting each pair of neighboring segments, the intermediate hinge being located between the central passage and the channel, and the periphery hinge being located at the exterior surface of the segments;
wherein one or more electrical cables extending between the distal tip and the handle are arranged in the channel.

2. The endoscope according to claim 1, wherein a bending cover is arranged on the outer surface of the bending section and is configured to cover the recess to keep the one or more electrical cables in the channel.

3. The endoscope according to claim 1 or 2, wherein lips are arranged on at least two segments, the lips being arranged to extend from a transition between the exterior surface of the segments and the recess, and to follow a circumferential outer periphery of the segment.

4. The endoscope according to claim 3, wherein the lips being configured such that the width of an opening to the recess is smaller than a maximum width of the recess.

5. The endoscope according to claim 4, wherein at least one lip on one segment is extending from a first side of the recess and at least one lip on another segment is extending from a second opposite side of the recess.

6. The endoscope according to any one of the previous claims, wherein the intermediate hinge and the periphery hinge are placed on opposite sides of the central passage.

7. The endoscope according to any one of the previous claims, wherein at least one segment has a film of material covering the recess, where the film is in one piece of material with the bending section, and the film has a thickness smaller than a thickness of a hinge.

8. The endoscope according to any one of the previous claims, wherein a cross-sectional area of the electrical cables is between 10 % and 85 % of a cross-sectional area of the recess.

9. The endoscope according to any one of claims 1-7, wherein the cross-sectional area of the electrical cables is between 10 % and 65 % of the cross-sectional area of the recess.

10. The endoscope according to any one of the previous claims, wherein a working channel tube is arranged in the central passage.

11. The endoscope according to any one of the previous claims, wherein the number of hinges between two segments is exactly two.

12. The endoscope according to any one of the previous claims, wherein the circumferential wall has a varying thickness with a minimum thickness at a position connecting to a hinge, and a maximum thickness next to the recess.

13. The endoscope according to any one of the previous claims, wherein the intermediate hinge has a height, measured in a radial direction from a center axis, which is at least 25 % larger than the height of the periphery hinge.

14. A method for assembling an endoscope according to any one of the previous claims, comprising:
- providing a bending section molded in one piece from a fused polymer, and having:
• segments connected to each other by hinges, the hinges allowing two neighboring segments to bend relative to each other in a bending plane, an exterior surface of the segments forms an outer surface of the bending section,
• a passage passing each segment and extending from a distal end to a proximal end of the bending section, where each segment has a circumferential wall, the circumferential wall has an inner surface facing and encircling the passage,
• a recess extending from the exterior surface of the segments into the segments, forming a channel extending in parallel with the central passage through the segments of the bending section,
• an intermediate hinge and a periphery hinge are connecting each pair of neighboring segments, the intermediate hinge is placed between the central passage and the channel, and the periphery hinge is placed at the exterior surface of the segments,
- arranging a working channel tube in the passage,
- arranging electrical cables in the channel,
- arranging a bending cover on the outer surface of the bending section.

15. A system comprising an endoscope according to any one of claims 1-13, a monitor and a control unit.
